# EUROPEAN PATENT SPECIFICATION

(11) **EP 2 358 890 B1**
(45) Date of publication and mention of the grant of the patent: **27.03.2013**
(21) Application number: 09765048.5
(22) Date of filing: 10.12.2009
(51) Int. Cl.: C12P 7/64

(54) **Process for the production of bio-oil from biomass**
Verfahren zur Herstellung von Bioöl aus Biomasse
Procédé pour la production de bio-huile à partir de biomasse

(30) Priority: 18.12.2008 IT MI20082249
(43) Date of publication of application: 24.08.2011
(73) Proprietor: ENI S.p.A., 00144 Rome (IT)
(72) Inventor: BIANCHI, Daniele, I-20020 Arese (IT); ROMANO, Anna Maria, I-28100 Novara (IT); FRANZOSI, Giuliana, I-28100 Novara (IT); BOSETTI, Aldo, I-13100 Vercelli (IT)
(74) Representative: Maiwald Patentanwalts GmbH
(86) International application number: PCT/EP2009/008862
(87) International publication number: WO 2010/069516

(56) References cited:
- WO-A1-2010/015404
- WO-A2-2010/046051
- DAI ET AL: "Biodiesel generation from oleaginous yeast Rhodotorula glutinis with xylose assimilating capacity", AFRICAN JOURNAL OF BIOTECHNOLOGY, vol. 6, 2007, pages 2130-2134, XP008101992, cited in the application
- MARZIALETTI ET AL: "Dilute acid hydrolysis of loblolly pine: a comprehensive approach", INDUSTRIAL AND ENGINEERING CHEMICAL RESEARCH, vol. 47, August 2008 (2008-08), pages 7131-7140, XP002553893,
- EL-FADALY ET AL: "Conversion of corn protelan to microbial oil by Rhodotorula glutinis", JOURNAL OF AGRICULTURAL SCIENCES, MANSOURA UNIVERSITY, vol. 33, January 2008 (2008-01), pages 5271-5281, XP002553847,
- LI ET AL: "Optimization of culture conditions for lipid production by Rhodosporidium toruloides", CHINESE JOURNAL OF BIOTECHNOLOGY, vol. 22, 2006, pages 650-656, XP022857389,
- MOYA ET AL: "Fermentation of acid hydrolysates from olive-tree pruning debris by Pachysolen tannophilus", BIOPROCESS AND BIOSYSTEMS ENGINEERING, vol. 31, March 2008 (2008-03), pages 611-617, XP019631489,
- ZHAO ET AL: "A new route to improved glucose yields in cellulose hydrolysis", AICHE 2006 Annual Meeting, San Francisco, California, USA, 2006, page 42, XP002634981, Retrieved from the Internet: URL:www.biorefinery.nl/fileadmin/biorefine ry/docs/AICHE_2006_Biorefineries.pdf [retrieved on 2011-05-02]
- FANTA ET AL: "Hydrolysis of wheat straw hemicellulose with trifluoroacetic acid. Fermentation of xylose with Pachysolen tannophilus", BIOTECHNOLOGY AND BIOENGINEERING, vol. XXVI, 1984, pages 1122-1125, XP002549080,

## Description

The present invention relates to a process for the production of bio-oil from biomass including at least one polysaccharide.

More specifically, the present invention relates to a process for the production of bio-oil from biomass including at least one polysaccharide which comprises subjecting said biomass to acid hydrolysis, subjecting the mixture obtained from said acid hydrolysis to enzymatic hydrolysis, subjecting the sugars obtained from said acid and enzymatic hydrolyses to fermentation in the presence of at least one oleaginous yeast, and subjecting the aqueous suspension comprising cells of said oleaginous yeast obtained from said fermentation to thermal treatment.

The bio-oil thus obtained can be advantageously used in the production of biofuels which can be used as such, or in a mixture with other fuels, for motor vehicles. Or, said bio-oil can be used as such (bio-combustible), or in a mixture with fossil combustibles (combustible oil, lignite, etc.), for the generation of electric energy.

Generally speaking, a biomass is any substance having an organic, vegetable or animal matrix, which can be destined for energy purposes, for example, as raw material for the production of biofuels and/or bio-combustibles, or components which can be added to fuels and/or combustibles. Biomass can, therefore, form a renewable energy source as an alternative to traditional raw materials of a fossil origin normally used in the production of fuels. For this purpose, lignocellulosic biomass is particularly useful.

The production of sugars from biomass, in particular from lignocellulosic biomass, is known in the art.

Lignocellulosic biomass is a complex structure comprising three main components: cellulose, hemicellulose and lignin. Their relative quantities vary according to the type of lignocellulosic biomass used. In the case of plants, for example, said quantities vary according to the species and age of the plant.

Cellulose is the greatest constituent of lignocellulosic biomass and is generally present in quantities ranging from 30% by weight to 60% by weight with respect to the total weight of the lignocellulosic biomass. Cellulose consists of glucose molecules (from about 500 to 10000 units) bound to each other through a β-1,4-glucoside bond. The establishment of hydrogen bonds between the chains causes the formation of crystalline domains which give resistance and elasticity to vegetal fibres. In nature, it can only be found in its pure state in annual plants such as cotton and flax, whereas in ligneous plants it is always accompanied by hemicellulose and lignin.

Hemicellulose which is generally present in a quantity ranging from 10% by weight to 40% by weight with respect to the total weight of the lignocellulosic biomass appears as a mixed polymer, relatively short (from 10 to 200 molecules) and branched, made up of both sugars with six carbon atoms (glucose, mannose, galactose) and sugars with five carbon atoms (xylose, arabinose). Some important properties of vegetal fibres are due to the presence of hemicellulose, of which the main property is that of favouring the imbibition of said vegetal fibres, when water is present, causing their swelling. Hemicellulose also has adhesive properties and, therefore, tends to harden or develop a horny consistency, when it dehydrates, with the consequence that said vegetal fibres become rigid and are imbibed more slowly.

Lignin is generally present in a quantity ranging from 10% by weight to 30% by weight with respect to the total weight of the lignocellulosic biomass. Its main function consists in binding and cementing the various vegetal fibres to each other, so as to give the plant compactness and resistance and also provides protection against insects, pathogen agents, lesions and ultraviolet light. It is mainly used as combustible, but is also currently widely used in industry as a disperser, hardener, emulsifying agent, for plastic laminates, cardboards and rubber products. It can also be chemically treated to produce aromatic compounds, like vanillin, syringaldehyde, p-hydroxybenzaldehyde, which can be used in pharmaceutical chemistry, or in the cosmetic and food industry.

In order to optimize the transformation of lignocellulosic biomass into products for energy use, subjecting said biomass to a preliminary treatment to separate the lignin and hydrolyze the cellulose and hemicellulose to simple sugars such as, for example, glucose and xylose, is known. Said sugars can be used as carbon sources in fermentation processes in the presence of micro-organisms for the production of alcohols and/or lipids.

Patent application US 2008/0102176, for example, describes a method for the extraction of vegetable fats comprising: pulverizing the raw material containing cellulose in order to obtain particles with a diameter of 1-2 mm; dipping the particles in sulfuric acid at a concentration equal to 1-2% to acidify said particles in order to increase the hydrolysis of the cellulose and to regulate the pH to a value of 4.5 ± 0.5; removing the particles acidified by the sulfuric acid and adding, in sequence, cellulase and an oleaginous yeast to the acidified particles and subjecting to fermentation for 8-9 days at a temperature of 25-30°C and a humidity of 85-90%; adding an aliphatic hydrocarbon as solvent to the fermentation products in order to extract the fats obtaining an extraction mixture; and removing the acidified particles remaining in the extraction mixture and separating the fats from the solvent by distillation obtaining raw oil. The cellulase is preferably *Trichoderma viride* and the oleaginous yeast is *Rhodotorula glutinis.* The fats obtained can be converted to biodiesel after esterification.

Dai et al. describe the production of biodiesel from oleaginous yeasts in the article: ""Biodiesel generation from oleaginous yeast Rhodotorula glutinis with xylose assimilating capacity", published in "African Journal of Biotechnology" (2007), Vol. 6 (18), pages 2130-2134. In this article, the lignocellulosic biomass is ground and subjected to acid hydrolysis in the presence of sulfuric acid. The sugars thus obtained, are used as carbon sources in a fermentation process in the presence of a previously selected strain of *Rhodotorula glutinis,* capable of also using pentoses, xylose in particular, with the purpose of obtaining oils which are subsequently extracted by Soxhlet extraction and subjected to transesterification in order to obtain biodiesel.

Various kinds of thermal treatments are also known in the art (such as, for example, liquefaction processes, hydrothermal treatments) which allow solid biomasses (such as for example, lignocellulosic biomasses, biomasses deriving from organic waste, agricultural waste and/or waste deriving from the agricultural industry, biomasses deriving from household waste) to be converted into liquid products in order to transform a voluminous material, which is difficult to handle and has a low energy density, into a bio-oil having a high energy density (i.e. a high calorific value) which can be used as such as biofuel and/or bio-combustible, or as a source of chemical products or a source of biofuels and/or bio-combustibles.

Greater details relating to thermal treatments (hydrothermal treatments or liquefaction treatments) of biomasses can be found, for example, in "Energy Sources, Part A: Recovery, Utilization and Environmental Effects", "Mechanisms of Thermochemical Biomass Conversion Processes. Part 3: Reactions of Liquefaction" (2008), 30:7, pages 649-659; or in "Energy & Environmental Science" (2008), "Thermochemical biofuel production in hydrothermal media: A review of sub- and supercritical water technologies", 1, pages 32-65.

The patent US 4,334,026 describes a bio-thermal hybrid liquefaction process for improving the liquefaction of compounds containing carbon for producing liquid fuels containing alcohol comprising: feeding organic compounds to a fermentation reactor; fermenting said organic compounds under conditions suitable for producing alcohol in a biological liquid culture; separating the liquid fuel containing alcohol and the fermentation residue, removing the water and introducing said fermentation residue into a thermochemical converter; thermochemically converting at least most of the organic component of said fermentation residue operating under high temperature conditions obtaining thermochemically converted products and thermochemical residues, a part of at least one of said thermochemically converted products or their derivatives, or of said thermochemical residues, being sent to said fermentation reactor. Land or water plants (e.g., algae), forest and/or agricultural waste, industrial waste, and the like, can be used as organic compounds.

Patent US 4,935,567 describes a process for the liquefaction of a biomass containing cellulose in order to obtain a liquefied product, which comprises: (a) feeding the biomass, water and an organic liquid containing oxygen (for example, an ester, a ketone, an alcohol) to a reaction vessel, obtaining a mixture which essentially consists of said biomass, said organic liquid and water, the total organic liquid containing oxygen and water in said mixture being equal to 5-20 parts by weight per parts by weight of biomass (dry weight) and the quantity of organic liquid containing oxygen in said mixture being equal to 5%-80% with respect to the total of said liquid and water; (b) heating said mixture to 200°C-400°C, at a pressure of 3-100 atmospheres, obtaining a liquefied product in the form of an oily liquid phase co-existing with an aqueous phase; (c) separating said oily liquid phase from said aqueous phase and recovering said oily liquid phase as liquefied product; and (d) heating said oily liquid phase separated to distill the compounds having a low boiling point and recovering the liquefied product having an increased calorific value as distillation residue. In order to accelerate the liquefaction, said liquefaction process can be carried out in the presence of an alkaline catalyst (for example, sodium hydroxide, potassium hydroxide, sodium carbonate, potassium carbonate, and the like). The liquefied product thus obtained can be used as fuel or as raw material for various chemical compounds.

The above processes, however, can have various drawbacks.

In order to hydrolyze both polysaccharide components of the biomass (e.g., hemicellulose and cellulose), for example, in particular in the presence of diluted sulfuric acid (e.g. at concentrations ranging from 0.5% to 11%), it is necessary to operate at high temperatures (e.g., higher than 160°C). At these temperatures, there is the formation of by-products deriving from the dehydration of the sugars and from the partial depolymerization of the lignin. These by-products, in particular furfural, hydroxymethylfur-fural, phenolic compounds, can act as growth inhibitors of the microorganisms normally used in the subsequent sugar fermentation processes. Furthermore, in the presence of diluted sulfuric acid, the sugar yield, in particular glucose, is normally low (e.g., lower than 50%).

Furthermore, before subjecting the sugars to fermentation, it may be necessary to neutralize the sulfuric acid in order to obtain pH values acceptable for the subsequent fermentation. The neutralization is generally carried out by adding oxides and/or hydroxides such as, for example; calcium oxide, calcium hydroxide, or barium hydroxide, with the consequent formation of salts (e.g., calcium sulfate, calcium sulfate dihydrate ("gypsum"), barium sulfate) which precipitate and must therefore be separated from the sugars before the latter are subjected to fermentation. In addition, these salts must be subsequently disposed of by means of suitable treatment with a consequent increase in the production costs. Furthermore, it is onerous and extremely difficult to recover and reuse said inorganic acid.

The presence of alcohol in the liquid fuels produced by liquefaction can also cause difficulty in the separation of said liquid fuels from the aqueous starting biomass due to the high polarity of said liquid fuels. Furthermore, the use of an organic liquid (for example, ester, ketone, alcohol) in the liquefaction process can create difficulty in the separation phase of the oily liquid phase from the aqueous phase due to the presence of said organic liquid, in addition to an increase in the production costs.

The Applicant has now found that the production of bio-oil from biomass (i.e. the conversion of the biomass into liquid products which can be used, for example, as bio-combustibles and/or biofuels), in particular biomass including at least one polysaccharide, can be advantageously carried out by means of a process which comprises subjecting said biomass to acid hydrolysis in the presence of an aqueous solution of at least one organic acid, selected from alkyl- or aryl-sulfonic acid having from C₇ to C₂₀ carbon atoms, or from halogenated carboxylic acids at a temperature ranging from 80°C to 160°C, subjecting the mixture obtained from said acid hydrolysis to enzymatic hydrolysis, subjecting the sugars obtained from said acid hydrolysis and enzymatic hydrolysis to fermentation in the presence of at least one oleaginous yeast, and subjecting the aqueous suspension comprising cells of said oleaginous yeast obtained from said fermentation to thermal treatment at a temperature ranging from 150°C to 330°C.

Numerous advantages are obtained by means of this process. For example, said process allows to obtain a high yield of pentose and hexose sugars, deriving from the acid hydrolysis of said biomass, which can be subsequently used as carbon sources in fermentation processes of oleaginous yeasts for the production of bio-oil. The bio-oil thus obtained can be advantageously used in the production of biofuels, which can be used used in the production of biofuels, which can be used as such, or mixed with other fuels, for motor vehicles. Or, said bio-oil can be used as such (bio-combustible), or in a mixture with fossil bio-combustibles (combustible oil, lignite, etc.), for the generation of electric energy or heat.

Furthermore, the possibility of operating at temperature which are not high, i.e. lower than or equal to 160°C, allows to reduce the formation of by-products, such as, for example, furfural, hydroxymethyl furfural, phenolic compounds, which can act as growth inhibitor of micro-organisms normally used in the subsequent fermentation processes of sugars.

Another important advantage lies in the fact that said process allows the organic acid to be recovered, which can then be recycled to the above process, in particular, to the acid hydrolysis of the biomass. Said recovery also allows to avoid the neutralization step and, therefore, the production of salts and their subsequent disposal.

A further significant advantage lies in the fact that the aqueous phase which is obtained after thermal treatment of the aqueous suspension comprising cells of said oleaginous yeast, said aqueous phase comprising sugars, glucoside and protein fractions soluble in water, deriving from the thermal treatment (thermal hydrolysis) of the proteins and polysaccharides contained in the cellular membrane of the oleaginous yeasts used, can be recycled to the above process, in particular it can be recycled to the fermentation. In this way, it is possible to reduce the quantity of nitrogen which must normally be added to the culture medium in which the fermentation takes place and to provide additional sugars for the fermentation.

An object of the present invention therefore relates to a process for the production of bio-oil from biomasses, including at least one polysaccharide, comprising:
- subjecting said biomass to acid hydrolysis in the presence of an aqueous solution of at least one organic acid selected from alkyl- or aryl-sulfonic acids having from C₇ to C₂₀ carbon atoms, preferably from C₉ to C₁₅ carbon atoms, or from halogenated carboxylic acids, at a temperature ranging from 80°C to 160°C, preferably from 100°C to 150°C, obtaining a first mixture comprising a first solid phase and a first aqueous phase;
- subjecting said first mixture to enzymatic hydrolysis obtaining a second mixture comprising a second solid phase and a second aqueous phase;
- subjecting said second aqueous phase to fermentation in the presence of at least one oleaginous yeast obtaining an aqueous suspension comprising cells of said oleaginous yeast;
- subjecting said aqueous suspension to thermal treatment obtaining an oily phase comprising bio-oil and a third aqueous phase wherein said thermal treatment is carried out at a temperature ranging from 150°C to 330°C.

For the purposes of the present description and of the following claims, the definition of the numerical ranges always include extremes unless otherwise specified.

In accordance with a preferred embodiment of the present invention, said polysaccharide can be selected from cellulose, hemicellulose, or mixtures thereof. Cellulose, or mixtures of hemicellulose and cellulose, are particularly preferred.

In accordance with a further preferred embodiment of the present invention, said biomass is a lignocellulosic biomass. As already mentioned above, lignocellulosic biomass includes three components: hemicellulose, cellulose and lignin.

Said lignocellulosic biomass is preferably selected from:
- the products of crops expressly cultivated for energy use (for example, miscanthus, millet, common cane), comprising by-products, residues and waste of said crops or of their processing;
- the products of agricultural cultivations, forestation and silviculture, comprising wood, plants, residues and by-products of agricultural processing, of forestation and of silviculture;
- the agri-foodstuffs by-products intended for human or animal feeding;
- the non-chemically treated residues of the paper industry;
- the waste coming from the differentiated collection of solid urban waste (e.g. urban waste of a vegetable origin, paper, etc.).

In accordance with a preferred embodiment of the present invention, said biomass can be treated by subjecting it to a preliminary milling or sizing process before being subjected to acid hydrolysis. Said biomass is preferably treated until particles having a diameter ranging from 0.1 mm to 10 mm, more preferably ranging from 0.5 mm to 4 mm, are obtained. Particles having a diameter lower than 1 mm are particularly preferred.

According to a preferred embodiment of the present invention, said biomass is present in the reaction mixture in a quantity ranging from 5% by weight to 40% by weight, preferably from 20% by weight to 35% by weight, with respect to the total weight of the reaction mixture.

For the purposes of the present description and of the following claims, the term "reaction mixture" means the mixture comprising the biomass and the aqueous solution of said at least one organic acid as defined above.

In accordance with a preferred embodiment of the present invention, said at least one organic acid is soluble in water and extractable with an organic solvent insoluble in water.

For the purposes of the present invention and of the following claims, the term "organic acid soluble in water" means an organic acid having a solubility in distilled water, at 25°C, of at least 0.5 g/100 ml of distilled water, preferably at least 2 g/100 ml of distilled water.

For the purposes of the present invention and of the following claims, the term "organic acid extractable with an organic solvent insoluble in water" refers to an organic acid which can be extracted with an organic solvent insoluble in water with a yield of at least 80%, preferably at least 90%, said yield being calculated with respect to the total quantity of organic acid present in the aqueous solution.

For the purposes of the present invention and of the following claims, the term "organic solvent insoluble in water" refers to an organic solvent which has a solubility in distilled water, at 25°C, lower than 4% by volume, preferably lower than 2% by volume.

According to a preferred embodiment of the present invention, said alkyl- or aryl-sulfonic acids can be selected from: dodecyl-sulfonic acid, para-toluene-sulfonic acid, 1-naphthalene-sulfonic acid, 2-naphthalene-sulfonic acid, 1,5-naphthalene-disulfonic acid, or mixtures thereof. Para-toluene-sulfonic acid, 2-naphthalene-sulfonic acid, 1,5-naphthalene-disulfonic acid, or mixtures thereof, are particularly preferred.

According to a preferred embodiment of the present invention, said halogenated carboxylic acids can be selected from those having a number of carbon atoms not higher than 20, preferably from 2 to 15, such as, for example, trifluoroacetic acid, dichloroacetic acid, trichloroacetic acid, perfluoro-octanoic acid, or mixtures thereof. Dichloroacetic acid, trichloroacetic acid, perfluoro-octanoic acid, or mixtures thereof, are particularly preferred.

According to a preferred embodiment of the present invention, said at least one organic acid is present in the aqueous solution at a concentration of 0.1% by weight to 5% by weight, preferably from 0.5% by weight to 2.5% by weight, with respect to the total weight of the aqueous solution.

Said organic acid acts as catalyst for the acid hydrolysis of said biomass. In particular, when the starting biomass is a lignocellulosic biomass, said organic acid specifically acts as catalyst for the acid hydrolysis of the hemicellulose. It should be noted that the process object of the present invention, when the starting biomass is a lignocellulosic biomass, not only allows the acid hydrolysis of the hemicellulose to be obtained, but also allows to improve the disposition of the cellulose, said cellulose remaining substantially non-hydrolyzed, for the subsequent enzymatic hydrolysis, thanks to an improved destructuring of the starting biomass.

According to a preferred embodiment of the present invention, said acid hydrolysis can be carried out for a time ranging from 20 minutes to 6 hours, preferably from 30 minutes to 3 hours.

Said acid hydrolysis can be carried out in reactors known in the art, such as, for example, autoclaves, or suitable extruders.

According to a preferred embodiment of the present invention, said first solid phase comprises lignin and cellulose and said first aqueous phase comprises at least one sugar having from C₅ to C₆ carbon atoms and said at least one organic acid. Said sugar is preferably xylose. Said xylose preferably derives from the acid hydrolysis of the hemicellulose.

Said acid hydrolysis allows at least one sugar having from C₅ to C₆ carbon atoms to be obtained, in particular xylose deriving from the acid hydrolysis of the hemicellulose, with a high yield. More specifically, said acid hydrolysis allows a yield of xylose higher than or equal to 80% to be obtained, said yield being calculated with respect to the total quantity of xylose present in the starting biomass.

Said acid hydrolysis also allows high yields of cellulose and lignin to be obtained.

In order to recover said at least one organic acid, said first mixture can be subjected to extraction with an organic solvent insoluble in water.

According to a preferred embodiment of the present invention, said process also comprises subjecting said first mixture to extraction with at least one organic solvent insoluble in water. Said organic solvent insoluble in water can be preferably selected from: halogenated hydrocarbons such as, for example, methylene chloride, monochlorobenzene, dichlorobenzene, or mixtures thereof; aromatic hydrocarbons such as, for example, toluene, xylene, or mixtures thereof; C₄-C₆ aliphatic alcohols such as n-butanol, n-pentanol, or mixtures thereof. Mixtures comprising at least one C₄-C₆ aliphatic alcohol (e.g., butanol) and at least one aromatic hydrocarbons (e.g., toluene), are particularly preferred.

Said organic solvent insoluble in water is subsequently evaporated obtaining a further solid phase comprising said at least one organic acid.

As specified above, the process object of the present invention, allows said at least one organic acid to be recovered with a high yield, in particular, with a yield of at least 80%, preferably of at least 90%, said yield being calculated with respect to the total quantity of organic acid present in said aqueous solution. Said at least one organic acid can therefore be subsequently recycled according to the process object of the present invention, in particular it can be recycled to the acid hydrolysis. It should be noted that, when the recovery of said at least one organic acid is equal to 100%, said first aqueous phase will be free of said at least one organic acid. Furthermore, the organic solvent evaporated, after condensation, can also be recycled to the process of the present invention, in particular it can be recycled to said extraction.

According to a preferred embodiment of the present invention, said process also comprises recycling said at least one organic acid to said acid hydrolysis.

According to a preferred embodiment of the present invention, said process also comprises recycling said at least one organic solvent insoluble in water to said extraction.

For the purposes of the process object of the present invention, said enzymatic hydrolysis can be carried out according to techniques known in the art as described, for example in United States Patents US 5,628,830, US 5,916,780 e US 6,090,595, using commercial enzymes such as for example, Celluclast 1.5L (Novozymes), Econase CE (Rohm Enzymes), Spezyme (Genecor), Novozym 188 (Novozymes), used individually or mixed with each other.

In order to adjust the pH to values ranging from 4 to 6, preferably from 4.5 to 5.5, glacial acetic acid can be added to said first mixture, before the enzymatic hydrolysis, in such a quantity so as to obtain the desired pH.

According to a preferred embodiment of the present invention, said second solid phase comprises lignin and said second aqueous phase comprises at least one sugar having from C₅ to C₆ carbon atoms, preferably xylose, glucose, cellobiose, or mixtures thereof. In particular, said second aqueous phase comprises xylose deriving from the acid hydrolysis of hemicellulose, glucose and cellobiose deriving from the enzymatic hydrolysis of cellulose.

In particular, this enzymatic hydrolysis allows a high glucose yield to be obtained, more specifically a glucose yield higher than or equal to 90%, said yield being calculated with respect to the total quantity of glucose present in the starting biomass.

The quantity of sugars contained in the starting biomass as also the quantity of sugars obtained after hydrolysis (acid hydrolysis and/or enzymatic hydrolysis), can be determined by means of techniques known in the art such as, for example, High Performance Liquid Chromatography (HPLC).

Said second solid phase and said second aqueous phase, can be separated by techniques known in the art such as, for example, filtration, centrifugation. Said phases are preferably separated by filtration.

Said second solid phase, comprising lignin, can be upgraded as combustible, for example as combustible for producing the energy necessary for sustaining the treatment processes of the biomass.

According to a preferred embodiment of the present invention, said fermentation can be carried out at a temperature ranging from 20°C to 40°C, preferably from 25°C to 35°C.

According to a preferred embodiment of the present invention, said fermentation can be carried out for a time ranging from 3 days to 10 days, preferably from 4 days to 8 days.

According to a preferred embodiment of the present invention, said fermentation can be carried out at a pH ranging from 4.5 to 6.5, preferably from 5 to 6. In order to maintain the pH within the desired ranges, an aqueous solution of at least one inorganic base such as, for example, sodium hydroxide, potassium hydroxide, calcium hydroxide, magnesium hydroxide, or mixtures thereof, can be added to the culture medium used for the fermentation, in a quantity which is such as to obtain the desired pH.

According to a preferred embodiment of the present invention, said oleaginous yeast can be selected from: *Rhodotorula glutinis, Rhodotorula gracilis, Rhodotorula graminis, Lypomices starkeyi, Lypomices lipofer, Trigonopsis variabilis, Candida kefyr, Candida curvata, Candida lipolytica, Torulopsis sp., Pichia stipitis.*

According to a preferred embodiment of the present invention, said fermentation is a fed-batch fermentation.

Before being used in said fermentation, said oleaginous yeast, is preferably grown in a culture medium containing xylose, cellobiose, glucose, or mixtures thereof, at a concentration preferably ranging from 1% by weight to 2% by weight, with respect to the total weight of said culture medium.

Said fermentation can be advantageously carried out in fermenters known in the art, in the presence of culture mediums normally used for the purpose comprising various nutrients such as, for example, nitrogen, potassium phosphate, magnesium, salts, vitamins.

In order to concentrate the yeast cells in the aqueous suspension obtained from the fermentation, said aqueous suspension, before being subjected to thermal treatment, can be subjected to thickening treatment.

According to a preferred embodiment of the present invention, at the end of the fermentation, said aqueous suspension can be sent to a thickening treatment. In this phase, the concentration of the yeast cells is brought to values ranging from 5% by weight to 50% by weight, preferably from 15% by weight to 30% by weight, with respect to the total weight (dry weight) of the aqueous suspension. Said thickening can be carried out using techniques known in the art, such as, for example, sedimentation, decanting, flocculation, filtration, and the like.

Said thermal treatment (hydrothermal treatment) provides for the heating of the concentrated aqueous suspension deriving from the thickening treatment in a vessel, under pressure. Said thermal treatment can be carried out operating in different ways such as, for example, "batch", "semi-batch", or in continuous.

According to the present invention, said thermal treatment is carried out at a temperature ranging from 150°C to 330°C.

The pressure is preferably maintained at values which are such as to keep at least part of the water in said aqueous suspension in liquid state.

According to a preferred embodiment of the present invention, said thermal treatment can be carried out at a pressure ranging from 0.1 MPa to 25 MPa, preferably from 0.5 MPa to 18 MPa.

According to a preferred embodiment of the present invention, said thermal treatment can be carried out for a time equal to or higher than 1 minute, preferably ranging from 0.5 hours to 2 hours.

As the thermal energy necessary for the thermal treatment can derive either totally or partially from thermal recovery or from the combustion of traditional energy vectors, for example methane gas, LPG, mineral oil, coal, etc., the possibility of the thermal energy deriving from solar energy is not excluded.

During said thermal treatment, the breakage of the cellular membranes of the cells of the oleaginous yeast present in said aqueous suspension also takes place, obtaining a mixture including an oily phase comprising bio-oil and an aqueous phase comprising sugars, glucoside fractions and proteins soluble in water, deriving from the thermal treatment (thermal hydrolysis) of the proteins and polysaccharides contained in the cellular membrane of the oleaginous yeasts used. It should be noted that part of said proteins and of said polysaccharides, during said thermal treatment, can be converted to bio-oil.

According to a preferred embodiment of the present invention, at the end of said thermal treatment, the mixture obtained (oily phase + aqueous phase) can be cooled to a temperature ranging from 40°C to 90°C, preferably from 45°C to 80°C, before being fed to a separation/recovery section in order to separate the two phases.

Said separation is generally carried out by means of techniques known in the art. It can be advantageously carried out, for example, by means of static separation, or by extraction with at least one organic solvent insoluble in water which can be selected, for example, from methylene chloride, or refinery hydrocarbon cuts, obtaining an oily phase comprising bio-oil and a third aqueous phase. Alternatively, said separation can be carried out by means of fractionated distillation.

Said third aqueous phase is optionally further cooled to a temperature ranging from room temperature (25°C) to 50°C, and recycled to the fermentation. If impurities are still present (for example, traces of bio-oil, traces of inhibitors, etc.), said aqueous phase can be subjected to a detoxification treatment which can be carried out, for example, by filtration, centrifugation, or extraction with an organic solvent insoluble in water (e.g., a mixture of toluene and n-butanol).

During said thermal treatment, a gaseous phase is also formed, equal to about 10%-25% by weight with respect to the weight (dry weight) of the yeast cells present in said aqueous suspension, said gaseous phase comprising about 80% - 95% in moles of carbon dioxide, and from 10% in moles to 20% in moles of hydrocarbons having from 1 to 3 carbon atoms. Said gaseous phase, after separation, which can be carried out for example by depressurizing the pressurized vessel in which said thermal treatment is carried out, before sending the mixture obtained (oily phase + aqueous phase) after said thermal treatment, to separation, is generally upgraded into its hydrocarbon component as combustible gas. Alternatively, the separation of the gaseous phase can be carried out during the separation of the oily phase from the aqueous phase.

The process object of the present invention allows the bio-oils to be recovered with a yield ranging from 50% to 95%, preferably from 55% to 85%, said yield being calculated with respect to the total weight (dry weight) of the cells of oleaginous yeast present in the aqueous suspension obtained after fermentation.

The bio-oil obtained by means of the above process, can be sent to the subsequent processing phases in order to transform it, for example, into biofuel by means of treatment known in the art such as, for example, hydrogenation or cracking.

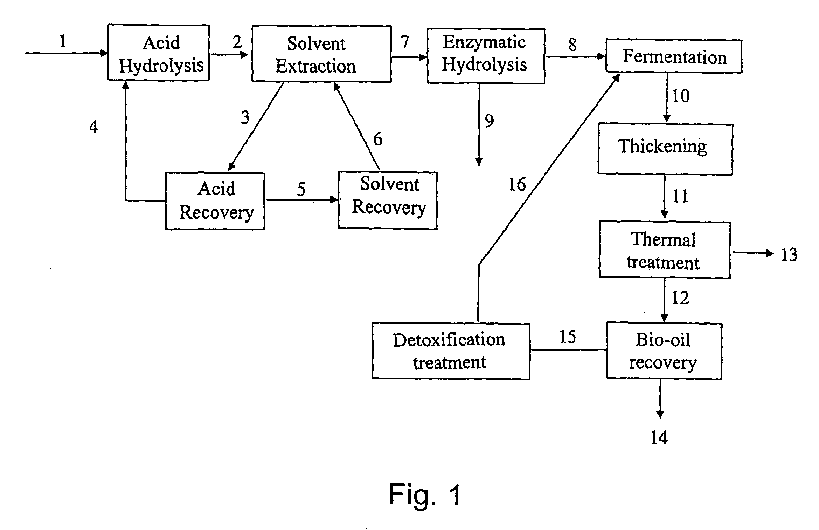

The present invention will now be described through an illustrative form with reference to Figure 1 provided hereunder.

According to a typical embodiment of the process object of the present invention, the biomass (line 1) (e.g., previously milled lignocellulosic biomass) is subjected to acid hydrolysis in the presence of an organic acid (e.g., 2-naphthalenesulfonic acid), obtaining a first mixture (line 2) comprising a first aqueous phase including xylose deriving from the hydrolysis of hemicellulose and said organic acid, and a first solid phase comprising lignin and cellulose.

As represented in Figure 1, said first mixture (line 2) is subjected to extraction with an organic solvent insoluble in water (e.g., a mixture of toluene and n-butanol) in order to recover said organic acid (line 3) and to recycle it, after evaporation of the organic solvent, to said acid hydrolysis (line 4). Said evaporated organic solvent (line 5), is condensed, recovered and recycled to said extraction (line 6).

After extraction with the organic solvent, said first mixture (line 7) is subjected to enzymatic hydrolysis, in the presence of an enzyme (e.g., an aqueous solution of Celluclast 1.5L and Novozym 188) obtaining a second mixture comprising a second aqueous phase including xylose deriving from the acid hydrolysis of hemicellulose, glucose and cellobiose deriving from the enzymatic hydrolysis of cellulose, and a second solid phase including lignin.

Before being subjected to enzymatic hydrolysis, glacial acetic acid can be added to said first mixture (line 7) in order to regulate the pH to values ranging from 4 to 6 (not represented in Figure 1).

Said second aqueous phase (line 8) and said second solid phase (line 9) are separated by filtration (not represented in Figure 1).

Said second aqueous phase (line 8) is subjected to fermentation in the presence of an oleaginous yeast (e.g., *Lypomyces starkey* NRRL 1389).

At the end of the fermentation, is obtained an aqueous suspension comprising cells of said oleaginous yeast (line 10) which is subjected to a thickening treatment in order to concentrate the cells of said oleaginous yeast in said aqueous suspension so as to obtain concentration values preferably ranging from 5% by weight to 30% by weight, with respect to the total weight (dry weight) of said aqueous suspension.

At the end of the thickening treatment, the concentrated aqueous suspension (line 11) is subjected to thermal treatment operating at a temperature, pressure and residence times which allow to obtain a mixture comprising an oily phase including bio-oil and a third aqueous phase comprising sugars, glucoside fractions and proteins soluble in water, deriving from the thermal treatment (thermal hydrolysis) of the proteins and polysaccharides contained in the cellular membrane of the oleaginous yeasts used, and possible impurities (for example, traces of bio-oil).

During the thermal treatment, a gaseous phase is also produced, comprising CO₂ and gaseous hydrocarbons having from 1 to 3 carbon atoms which can be separated, for example, by depressurizing the pressurized vessel in which said thermal treatment is carried out, before sending the mixture obtained (oily phase + aqueous phase) after said thermal treatment to the phase separation section. The gaseous phase thus obtained (line 13) can be sent to further treatment in order to upgrade its hydrocarbon component as combustible gas.

The mixture (oily phase + aqueous phase) obtained after thermal treatment is cooled by passing it through heat exchangers (not represented in Figure 1) and sent (line 12) to a phase separation section in order to separate and extract the two phases obtaining bio-oil and an aqueous phase.

The bio-oil (line 14) can be sent to the subsequent processing phases to transform it, for example, into biofuel by means of treatment, for example, hydrogenation or cracking (not illustrated in Figure 1).

As represented in Figure 1, the aqueous phase (line 15) is sent to detoxification treatment and is subsequently recycled, after possible further cooling into a heat exchanger (not represented in Figure 1), to the fermentation (line 16).

Some illustrative and non-limiting examples are below provided for a better understanding of the present invention and for its embodiment.

### EXAMPLE 1

### Acid hydrolysis of the biomass

300 g of coniferous wood previously milled (particle diameter < 1 mm) were added to a solution of 20 g of 2-naphthalenesulfonic acid in 1 1 of water.

The composition of the starting biomass was the following: 50% by weight of cellulose, 25% by weight of hemicellulose, 25% by weight of lignin, with respect to the total weight of the starting biomass.

The reaction mixture thus obtained, was maintained, under stirring, in an autoclave, at 140°C, for 1 hour, obtaining a first mixture comprising 225 g (dry weight) of a first solid phase (comprising cellulose and lignin) and 1095 g of a first aqueous phase (comprising xylose deriving from the hydrolysis of hemicellulose and 2-naphthalenesulfonic acid).

After cooling to 25°C, said first mixture was subjected to extraction with 1 l of a mixture of toluene and n-butanol.

The mixture of toluene and n-butanol was subsequently separated and evaporated, at reduced pressure, obtaining a further solid phase comprising 19.6 g (dry weight) of 2-naphthalenesulfonic acid (recovery yield 98% calculated with respect to the total quantity of the acid present in the aqueous solution).

After separation of the acid, glacial acetic acid was added to said first mixture until a pH of said first aqueous phase equal to 5 was obtained.

An aqueous solution of the enzyme Celluclast 1.5L (Novozymes) corresponding to 1485 FPU (Filter Paper Units) and the enzyme Novozym 188 (from Novozymes) corresponding to 18000 BGU (Beta Glucanase Units) was subsequently added. The suspension thus obtained was maintained under stirring, for 72 hours, at 45°C.

At the end of the enzymatic hydrolysis, 90 g (dry weight) of a second solid phase comprising lignin (75 g - dry weight) and non-hydrolyzed cellulose (15 g - dry weight) and 1200 g of a second aqueous phase comprising glucose (152 g - dry weight) and xylose (72 g - dry weight), were separated by filtration.

### Fermentation

250 ml of said second aqueous phase obtained as described above, containing 200 g/l of sugars were suitably diluted with distilled water until a solution having a final concentration of sugars equal to 50 g/l was obtained.

The following nutrients were added to this solution: ammonium sulfate 0.1%, KH₂PO₄ 0.1%, MgSO₄·7H₂O 0.005%, NaCl 0.001%, CaCl₂ 0.001%, yeast extract 0.1%. The fermentation broth (culture medium) thus obtained was inoculated in a fermenter with yeast cells *(Lypomices starkey* NRRL 1389) (inoculum equal to 2.5 g/l - dry weight) for a fermentation volume equal to 1 litre and the pH was maintained at 5.5 by the addition of an aqueous solution of NaOH 0.1 M.

The yeast cells were grown, under stirring at 500 rpm, at 30°C, aeration 0.5 l/min of sterile air.

A further 250 ml of said second aqueous phase were added to the fermenter, obtained from the hydrolysis of the biomass, containing 200 g/l of sugars, suitably diluted with distilled water to obtain a solution having a final concentration of sugars equal to 50 g/l.

### Thermal treatment

The aqueous suspension obtained, equal to about 1.25 1, comprising 52 g of yeast cells, was concentrated by centrifugation at 7000 rpm, for 20 minutes obtaining 0.25 1 comprising 20% (dry weight) of yeast cells which were placed in a 0.5 1 steel autoclave.

The autoclave was subjected to a nitrogen atmosphere and the internal temperature was rapidly brought to 315°C: the whole mixture was maintained, under stirring, at said temperature, for 1 hour. The maximum pressure registered in the autoclave was 11 MPa.

The autoclave was subsequently rapidly brought to a temperature equal to 70°C, and depressurized in order to remove the gaseous phase formed. Said gaseous phase, equal to about 6.2 g, was analyzed by gas chromatography online and was found to comprise 90% in moles of carbon dioxide.

The residual mixture (oily phase + aqueous phase) was fed to a separator funnel and extracted with methylene chloride obtaining an oily phase and a third aqueous phase.

Said oily phase was dried on sodium sulfate and subsequently evaporated at reduced pressure to remove the methylene chloride obtaining about 35 g of oil (yield on dry yeast cells equal to 67.3%).

Said aqueous phase, having a total COD equal to 43200 mg/l, was recycled to the fermentation.

## Claims

1. Process for the production of bio-oil from biomass including at least one polysaccharide comprising:
- subjecting said biomass to acid hydrolysis in the presence of an aqueous solution of at least one organic acid selected from alkyl- or aryl-sulfonic acids having from C₇ to C₂₀ carbon atoms, or from halogenated carboxylic acids, at a temperature ranging from 80°C to 160°C, obtaining a first mixture comprising a first solid phase and a first aqueous phase;
- subjecting said first mixture to enzymatic hydrolysis obtaining a second mixture comprising a second solid phase and a second aqueous phase;
- subjecting said second aqueous phase to fermentation in the presence of at least one oleaginous yeast obtaining an aqueous suspension comprising cells of said oleaginous yeast;
- subjecting said aqueous suspension to thermal treatment obtaining an oily phase comprising bio-oil and a third aqueous phase, wherein said thermal treatment is carried out at a temperature ranging from 150°C to 330°C.

2. Process according to claim 1, wherein said alkyl- or aryl sulfonic acids have from C₉ to C₁₅ carbon atoms.

3. Process according to claim 1 or 2, wherein said first temperature is ranging from 100°C to 150°C.

4. Process according to any one of the previous claims, wherein said polysaccharide is selected from cellulose, hemicellulose, or mixtures thereof.

5. Process according to any one of the previous claims, wherein said biomass is a lignocellulosic biomass.

6. Process according to claim 5, wherein said lignocellulosic biomass is selected from:
- the products of crops expressly cultivated for energy use, comprising by-products, residues and waste of said cultures or of their processing;
- the products of agricultural cultivations, forestation and silviculture, comprising wood, plants, residues and by-products of agricultural processings, of forestation and of silviculture;
- the agri-foodstuffs by-products intended for human or animal feeding;
- the non-chemically treated residues from the paper industry;
- the waste coming from the differentiated collection of solid urban waste.

7. Process according to any one of the previous claims, wherein said biomass is treated by subjecting it to a preliminary milling or sizing process before being submitted to acid hydrolysis.

8. Process according to claim 7, wherein said biomass is treated until obtaining particles having a diameter ranging from 0.1 mm to 10 mm.

9. Process according to any one of the previous claims, wherein said biomass is present in the reaction mixture in a quantity ranging from 5% by weight to 40% by weight with respect to the total weight of the reaction mixture.

10. Process according to any one of the previous claims, wherein said at least one organic acid is soluble in water and extractable with an organic solvent insoluble in water.

11. Process according to any one of the previous claims, wherein said alkyl- or aryl-sulfonic acids are selected from dodecyl-sulfonic acid, para-toluene-sulfonic acid, 1-naphthalene-sulfonic acid, 2-naphthalene-sulfonic acid, 1,5-naphthalene-disulfonic acid, or mixtures thereof.

12. Process according to any one of the previous claims, wherein said halogenated carboxylic acids are selected from those having a number of carbon atoms not higher than 20 such as, trifluoroacetic acid, dichloroacetic acid, trichloroacetic acid, perfluorooctanoic acid, or mixtures thereof.

13. Process according to any one of the previous claims, wherein said at least one organic acid is present in the aqueous solution at a concentration ranging from 0.1% by weight to 5% by weight with respect to the total weight of the aqueous solution.

14. Process according to any one of the previous claims, wherein said acid hydrolysis is carried out for a time ranging from 20 minutes to 6 hours.

15. Process according to any one of the previous claims, wherein said first solid phase comprises lignin and cellulose.

16. Process according to any one of the previous claims, wherein said first aqueous phase comprises at least one sugar having from C₅ to C₆ carbon atoms and said at least one organic acid.

17. Process according to any one of the previous claims, wherein said first mixture is submitted to extraction with an organic solvent insoluble in water.

18. Process according to claim 17, wherein said organic solvent insoluble in water is selected from: halogenated hydrocarbons such as methylene chloride, monochlorobenzene, dichlorobenzene, or mixtures thereof; aromatic hydrocarbons such as toluene, xylene, or mixtures thereof; C₄-C₆ aliphatic alcohols such as n-butanol, n-pentanol, or mixtures thereof.

19. Process according to any one of the previous claims, wherein said process comprises recycling said at least one organic acid in said acid hydrolysis.

20. Process according to any one of the previous claims, wherein said process comprises recycling said at least one organic solvent insoluble in water at said extraction.

21. Process according to any one of the previous claims, wherein said second solid phase comprises lignin.

22. Process according to any one of the previous claims, wherein said second aqueous phase comprises at least one sugar having from C₅ to C₆ carbon atoms.

23. Process according to any one of the previous claims, wherein said fermentation is carried out at a temperature ranging from 20°C to 40°C.

24. Process according to any one of the previous claims, wherein said fermentation is carried out for a time ranging from 3 days to 10 days.

25. Process according to any one of the previous claims, wherein said fermentation is carried out at a pH ranging from 4.5 to 6.5.

26. Process according to any one of the previous claims, wherein said oleaginous yeast is selected from: *Rhodotorula glutinis, Rhodotorula gracilis, Rhodotorula graminis, Lypomices starkeyi, Lypomices lipofer, Trigonopsis variabilis, Candida kefyr, Candida curvata, Candida lipolytica, Torulopsis sp., Pichia stipitis.*

27. Process according to any one of the previous claims, wherein said fermentation is a fed-batch fermentation.

28. Process according to any one of the previous claims, wherein said aqueous suspension, at the end of the fermentation, is sent to a thickening treatment wherein the concentration of the yeast cells is brought to values ranging from 5% by weight to 50% by weight with respect to the total weight (dry weight) of the aqueous suspension.

29. Process according to any one of the previous claims, wherein said thermal treatment is carried out at a pressure ranging from 0.1 MPa to 25 MPa.

30. Process according to any one of the previous claims, wherein said thermal treatment is carried out for a time equal to or higher than 1 minute.

31. Process according to any one of the previous claims, wherein said thermal treatment is carried out for a time ranging from 0.5 hours to 2 hours.

32. Process according to any one of the previous claims, wherein at the end of said thermal treatment, the obtained mixture (oily phase + aqueous phase) is cooled to a temperature ranging from 40°C to 90°C, before being fed to a section of separation/recovery of the following phases: said oily phase comprising bio-oil and said third aqueous phase.

33. Process according to claim 32, wherein said third aqueous phase is recycled to the fermentation.

## Patentansprüche

1. Verfahren zur Herstellung von Bioöl aus Biomasse mit mindestens einem Polysaccharid umfassend:
- Unterziehen der Biomasse einer Säurehydrolyse in Gegenwart einer wässrigen Lösung von mindestens einer organischen Säure ausgewählt aus Alkyl-oder Arylsulfonsäuren mit C₇ bis C₂₀ Kohlenstoffatomen, oder aus halogenierten Carbonsäuren, bei einer Temperatur im Bereich von 80°C bis 160°C, Erhalten einer ersten Mischung, die eine erste feste Phase und eine erste wässrige Phase umfasst;
- Unterziehen der ersten Mischung einer enzymatischen Hydrolyse, Erhalten einer zweiten Mischung, die eine zweite feste Phase und eine zweite wässrige Phase umfasst;
- Unterziehen der zweiten wässrigen Phase der Fermentation in Gegenwart von mindestens einer ölhaltigen Hefe, Erhalten einer wässrigen Suspension, die Zellen der ölhaltigen Hefe umfasst;
- Unterziehen der wässrigen Suspension einer thermischen Behandlung, Erhalten einer öligen Phase, die Bioöl und eine dritte wässrige Phase umfasst, wobei die thermische Behandlung bei einer Temperatur im Bereich von 150°C bis 300°C durchgeführt wird.

2. Verfahren nach Anspruch 1, wobei die Alkyl- oder Arylsulfonsäuren von C₉ bis C₁₅ Kohlenstoffatome haben.

3. Verfahren nach Anspruch 1 oder 2, wobei die erste Temperatur im Bereich von 100°C bis 150°C ist.

4. Verfahren nach einem der vorhergehenden Ansprüche, wobei das Polysaccharid aus Cellulose, Hemicellulose, oder Mischungen davon, ausgewählt ist.

5. Verfahren nach einem der vorhergehenden Ansprüche, wobei die Biomasse eine lignocellulosehaltige Biomasse ist.

6. Verfahren nach Anspruch 5, wobei die lignocellulosehaltige Biomasse ausgewählt ist aus:
- den Produkten der Kulturpflanzen, die ausdrücklich für den Energieeinsatz kultiviert werden, umfassend Nebenprodukte, Rückstände und Abfälle der Kulturen oder ihrer Verarbeitung;
- den Produkten der landwirtschaftlichen Kulturen, Aufforstung und Forstwirtschaft, umfassend Holz, Pflanzen, Rückstände und Nebenprodukte der landwirtschaftlichen Verarbeitungen, der Aufforstung und der Forstwirtschaft;
- den agrar-und ernährungswirtschaftlichen Nebenprodukten, die für die menschliche oder tierische Ernährung bestimmt sind;
- den nicht-chemisch behandelten Rückständen aus der Papierindustrie;
- dem Abfall, der aus der getrennten Sammlung von Siedlungsabfällen kommt.

7. Verfahren nach einem der vorhergehenden Ansprüche, wobei die Biomasse durch Unterziehen derselben eines vorläufigen Mahl- oder Dimensionierungsprozesses behandelt wird, bevor sie der Säurehydrolyse ausgesetzt wird.

8. Verfahren nach Anspruch 7, wobei die Biomasse bis zum Erhalt von Teilchen mit einem Durchmesser im Bereich von 0,1 mm bis 10 mm behandelt wird.

9. Verfahren nach einem der vorhergehenden Ansprüche, wobei die Biomasse in der Reaktionsmischung in einer Menge im Bereich von 5 Gew.% bis 40 Gew.%, bezogen auf das Gesamtgewicht der Reaktionsmischung, vorhanden ist.

10. Verfahren nach einem der vorhergehenden Ansprüche, wobei mindestens eine organische Säure in Wasser löslich und mit einem in Wasser unlöslichen organischen Lösungsmittel extrahierbar ist.

11. Verfahren nach einem der vorhergehenden Ansprüche, wobei die Alkyl-oder Arylsulfonsäuren aus Dodecylsulfonsäure, para-Toluolsulfonsäure, 1-Naphthalinsulfonsäure, 2-Naphthalinsulfonsäure, 1,5-Naphthalindisulfonsäure, oder Mischungen davon, ausgewählt sind.

12. Verfahren nach einem der vorhergehenden Ansprüche, wobei die halogenierten Carbonsäuren aus solchen ausgewählt sind, die eine Anzahl von nicht mehr als 20 Kohlenstoffatomen haben, wie Trifluoressigsäure, Dichloressigsäure, Trichloressigsäure, Perfluorooctanoic Säure, oder Mischungen davon.

13. Verfahren nach einem der vorhergehenden Ansprüche, wobei mindestens eine organische Säure in der wässrigen Lösung in einer Konzentration im Bereich von 0,1 Gew.% bis 5 Gew.%, bezogen auf das Gesamtgewicht der wässrigen Lösung, vorhanden ist.

14. Verfahren nach einem der vorhergehenden Ansprüche, wobei die Säurehydrolyse für eine Zeitdauer im Bereich von 20 Minuten bis 6 Stunden durchgeführt wird.

15. Verfahren nach einem der vorhergehenden Ansprüche, wobei die erste feste Phase Lignin und Cellulose umfasst.

16. Verfahren nach einem der vorhergehenden Ansprüche, wobei die erste wässrige Phase mindestens einen Zucker mit C₅ bis C₆ Kohlenstoffatomen und mindestens eine organische Säure umfasst.

17. Verfahren nach einem der vorhergehenden Ansprüche, wobei die erste Mischung einer Extraktion mit einem in Wasser unlöslichen organischen Lösungsmittel unterzogen wird.

18. Verfahren nach Anspruch 17, wobei das in Wasser unlösliche organische Lösungsmittel ausgewählt ist aus:
halogenierten Kohlenwasserstoffen, wie Methylenchlorid, Monochlorbenzol, Dichlorbenzol, oder Mischungen davon;
aromatischen Kohlenwasserstoffen, wie Toluol, Xylol, oder Mischungen davon;
C₄-C₆ aliphatischen Alkoholen, wie n-Butanol, n-Pentanol, oder Mischungen davon.

19. Verfahren nach einem der vorhergehenden Ansprüche, wobei das Verfahren Rückführen mindestens einer organischen Säure in die Säurehydrolyse umfasst.

20. Verfahren nach einem der vorhergehenden Ansprüche, wobei das Verfahren Rückführen mindestens eines in Wasser unlöslichen organischen Lösungsmittels bei der Extraktion umfasst.

21. Verfahren nach einem der vorhergehenden Ansprüche, wobei die zweite feste Phase Lignin umfasst.

22. Verfahren nach einem der vorhergehenden Ansprüche, wobei die zweite wässrige Phase mindestens einen Zucker mit C₅ bis C₆ Kohlenstoffatomen umfasst.

23. Verfahren nach einem der vorhergehenden Ansprüche, wobei die Fermentation bei einer Temperatur im Bereich von 20°C bis 40°C durchgeführt wird.

24. Verfahren nach einem der vorhergehenden Ansprüche, wobei die Fermentation für einen Zeitraum von 3 Tagen bis 10 Tagen durchgeführt wird.

25. Verfahren nach einem der vorhergehenden Ansprüche, wobei die Fermentation in einem pH-Bereich von 4,5 bis 6,5 durchgeführt wird.

26. Verfahren nach einem der vorhergehenden Ansprüche, wobei die ölhaltige Hefe ausgewählt ist aus:
*Rhodotorula glutinis, Rhodotorula gracilis, Rhodotorula graminis, Lypomices starkeyi, Lypomices lipofer, Trigonopsis variabilis, Candida kefyr, Candida curvata, Candida lipolytica, Torulopsis sp., Pichia stipitis.*

27. Verfahren nach einem der vorhergehenden Ansprüche, wobei die Fermentation eine Fed-Batch-Fermentation ist.

28. Verfahren nach einem der vorhergehenden Ansprüche, wobei die wässrige Suspension am Ende der Fermentation einer Verdickungsbehandlung unterzogen wird, wobei die Konzentration der Hefezellen auf Werte im Bereich von 5 Gew.% bis 50 Gew.% eingestellt wird, bezogen auf das Gesamtgewicht (Trockengewicht) der wässrigen Suspension.

29. Verfahren nach einem der vorhergehenden Ansprüche, wobei die thermische Behandlung bei einem Druck im Bereich von 0,1 MPa bis 25 MPa durchgeführt wird.

30. Verfahren nach einem der vorhergehenden Ansprüche, wobei die thermische Behandlung für eine Zeit, die gleich oder länger als 1 Minute ist, durchgeführt wird.

31. Verfahren nach einem der vorhergehenden Ansprüche, wobei die thermische Behandlung für eine Zeitdauer im Bereich von 0,5 Stunden bis 2 Stunden durchgeführt wird.

32. Verfahren nach einem der vorhergehenden Ansprüche, wobei am Ende der thermischen Behandlung die erhaltene Mischung (Ölphase + wässrige Phase) auf eine Temperatur im Bereich von 40°C bis 90°C abgekühlt wird, bevor sie zu einem Abschnitt der Trennung/Wiedergewinnung der folgenden Phasen zugeführt wird: ölige Phase umfassend Bioöl und dritte wässrige Phase.

33. Verfahren nach Anspruch 32, wobei die dritte wässrige Phase zur Fermentation zurückgeführt wird.

## Revendications

1. Procédé pour la production de bio-pétrole à partir d'une biomasse contenant au moins un polysaccharide, comprenant :
- la soumission de ladite biomasse à une hydrolyse acide en présence d'une solution aqueuse d'au moins un acide organique choisi parmi les acides alkyl- ou aryl-sulfoniques comportant de 7 à 20 atomes de carbone, ou parmi les acides carboxyliques halogénés, à une température située dans la plage allant de 80°C à 160°C, en obtenant un premier mélange comprenant une première phase solide et une première phase aqueuse ;
- la soumission dudit premier mélange à une hydrolyse enzymatique, en obtenant un deuxième mélange comprenant une deuxième phase solide et une deuxième phase aqueuse ;
- la soumission de ladite deuxième phase aqueuse à une fermentation en présence d'au moins une levure oléagineuse, en obtenant une suspension aqueuse comprenant des cellules de ladite levure oléagineuse ;
- la soumission de ladite suspension aqueuse à un traitement thermique, en obtenant une phase huileuse comprenant un bio-pétrole et une troisième phase aqueuse, ledit traitement thermique étant effectué à une température située dans la plage allant de 150°C à 330°C.

2. Procédé selon la revendication 1, dans lequel lesdits acides alkyl- ou aryl-sulfoniques comportent de 9 à 15 atomes de carbone.

3. Procédé selon la revendication 1 ou 2, dans lequel ladite première température est située dans la plage allant de 100°C à 150°C.

4. Procédé selon l'une quelconque des revendications précédentes, dans lequel ledit polysaccharide est choisi parmi la cellulose, l'hémicellulose, et leurs mélanges.

5. Procédé selon l'une quelconque des revendications précédentes, dans lequel ladite biomasse est une biomasse ligno-cellulosique.

6. Procédé selon la revendication 5, dans lequel ladite biomasse ligno-cellulosique est choisie parmi:
- les produits de cultures expressément cultivées pour un usage énergétique, y compris les sous-produits, les résidus et les rebuts desdites cultures ou de leur transformation ;
- les produits de cultures agricoles, de forestation et de sylviculture, y compris le bois, les plantes, les résidus et les sous-produits de transformations agricoles, de forestation et de sylviculture ;
- les sous-produits de l'industrie agroalimentaire destinés à l'alimentation humaine ou animale ;
- les résidus non traités chimiquement de l'industrie du papier ;
- les rebuts provenant du tri sélectif des ordures urbaines solides.

7. Procédé selon l'une quelconque des revendications précédentes, dans lequel ladite biomasse est traitée en étant soumise à un traitement préliminaire de broyage ou de calibrage avant d'être soumise à une hydrolyse acide.

8. Procédé selon la revendication 7, dans lequel ladite biomasse est traitée jusqu'à ce que soient obtenues des particules présentant un diamètre situé dans la plage allant de 0,1 mm à 10 mm.

9. Procédé selon l'une quelconque des revendications précédentes, dans lequel ladite biomasse est présente dans le mélange réactionnel en une quantité située dans la plage allant de 5 % en poids à 40 % en poids par rapport au poids total du mélange réactionnel.

10. Procédé selon l'une quelconque des revendications précédentes, dans lequel ledit au moins un acide organique est soluble dans l'eau et extractible avec un solvant organique insoluble dans l'eau.

11. Procédé selon l'une quelconque des revendications précédentes, dans lequel lesdits acides alkyl- ou aryl-sulfoniques sont choisis parmi l'acide dodécylsulfonique, l'acide paratoluènesulfonique, l'acide 1-naphtalènesulfonique, l'acide 2-naphtalènesulfonique, l'acide 1,5-naphtalènedisulfonique, et leurs mélanges.

12. Procédé selon l'une quelconque des revendications précédentes, dans lequel lesdits acides carboxyliques halogénés sont choisis parmi ceux ayant au plus 20 atomes de carbone, tels que l'acide trifluoroacétique, l'acide dichloroacétique, l'acide trichloroacétique, l'acide perfluorooctanoïque, ou leurs mélanges.

13. Procédé selon l'une quelconque des revendications précédentes, dans lequel ledit au moins un acide organique est présent dans la solution aqueuse à une concentration située dans la plage allant de 0,1 % en poids à 5 % en poids par rapport au poids total de la solution aqueuse.

14. Procédé selon l'une quelconque des revendications précédentes, dans lequel ladite hydrolyse acide est effectuée pendant un temps situé dans la plage allant de 20 minutes à 6 heures.

15. Procédé selon l'une quelconque des revendications précédentes, dans lequel ladite première phase solide comprend de la lignine et de la cellulose.

16. Procédé selon l'une quelconque des revendications précédentes, dans lequel ladite première phase aqueuse comprend au moins un sucre comportant 5 à 6 atomes de carbone et ledit au moins un acide organique.

17. Procédé selon l'une quelconque des revendications précédentes, dans lequel ledit premier mélange est soumis à une extraction avec un solvant organique insoluble dans l'eau.

18. Procédé selon la revendication 17, dans lequel ledit solvant organique insoluble dans l'eau est choisi parmi: les hydrocarbures halogénés tels que le chlorure de méthylène, le monochlorobenzène, le dichlorobenzène, ou leurs mélanges ; les hydrocarbures aromatiques tels que le toluène, le xylène, ou leurs mélanges; les alcools aliphatiques en C₄ à C₆ tels que le n-butanol, le n-pentanol, ou leurs mélanges.

19. Procédé selon l'une quelconque des revendications précédentes, lequel procédé comprend le recyclage dudit au moins un acide organique dans ladite hydrolyse acide.

20. Procédé selon l'une quelconque des revendications précédentes, lequel procédé comprend le recyclage dudit au moins un solvant organique insoluble dans l'eau lors de ladite extraction.

21. Procédé selon l'une quelconque des revendications précédentes, dans lequel ladite deuxième phase solide comprend de la lignine.

22. Procédé selon l'une quelconque des revendications précédentes, dans lequel ladite deuxième phase aqueuse comprend au moins un sucre comportant 5 à 6 atomes de carbone.

23. Procédé selon l'une quelconque des revendications précédentes, dans lequel ladite fermentation est effectuée à une température située dans la plage allant de 20°C à 40°C.

24. Procédé selon l'une quelconque des revendications précédentes, dans lequel ladite fermentation est effectuée pendant un temps situé dans la plage allant de 3 jours à 10 jours.

25. Procédé selon l'une quelconque des revendications précédentes, dans lequel ladite fermentation est effectuée à un pH situé dans la plage allant de 4,5 à 6,5.

26. Procédé selon l'une quelconque des revendications précédentes, dans lequel ladite levure oléagineuse est choisie parmi: *Rhodotorula glutinis, Rhodotorula gracilis, Rhodotorula graminis, Lypomices starkeyi, Lypomices lipofer, Trigonopsis variabilis, Candida kefyr, Candida curvata, Candida lipolytica, Torulopsis sp., Pichia stipitis.*

27. Procédé selon l'une quelconque des revendications précédentes, dans lequel ladite fermentation est une fermentation à écoulement discontinu.

28. Procédé selon l'une quelconque des revendications précédentes, dans lequel ladite suspension aqueuse, à la fin de la fermentation, est envoyée vers un traitement d'épaississement dans lequel la concentration des cellules de levure est portée à des valeurs situées dans la plage allant de 5 % en poids à 50 % en poids par rapport au poids total (poids sec) de la suspension aqueuse.

29. Procédé selon l'une quelconque des revendications précédentes, dans lequel ledit traitement thermique est effectué sous une pression située dans la plage allant de 0,1 MPa à 25 MPa.

30. Procédé selon l'une quelconque des revendications précédentes, dans lequel ledit traitement thermique est effectué pendant un temps égal ou supérieur à 1 minute.

31. Procédé selon l'une quelconque des revendications précédentes, dans lequel ledit traitement thermique est effectué pendant un temps situé dans la plage allant de 0,5 heure à 2 heures.

32. Procédé selon l'une quelconque des revendications précédentes, dans lequel, à la fin dudit traitement thermique, le mélange obtenu (phase huileuse + phase aqueuse) est refroidi à une température située dans la plage allant de 40°C à 90°C, avant d'être introduit dans une section de séparation/récupération des phases suivantes : ladite phase huileuse comprenant du bio-pétrole, et ladite troisième phase aqueuse.

33. Procédé selon la revendication 32, dans lequel ladite troisième phase aqueuse est recyclée vers la fermentation.
